(19) Europäisches Patentamt
European Patent Office
Office européen des brevets

(11) EP 0 566 138 B1

(12) **EUROPEAN PATENT SPECIFICATION**

(45) Date of publication and mention of the grant of the patent:
**18.11.1998 Bulletin 1998/47**

(51) Int Cl.6: **C07D 277/56**, C07D 417/12, A01N 43/78

(21) Application number: **93106230.1**

(22) Date of filing: **16.04.1993**

(54) **Amino thiazole derivatives and their use as fungicides**

Amino-Thiazolderivate und ihre Anwendung als Fungizide

Amino thiazoles et leur utilisation comme fongicides

(84) Designated Contracting States:
**DE ES FR GB IT NL PT**

(30) Priority: **17.04.1992 JP 122787/92**

(43) Date of publication of application:
**20.10.1993 Bulletin 1993/42**

(73) Proprietor: **HODOGAYA CHEMICAL CO., LTD.**
**Tokyo (JP)**

(72) Inventors:
- **Ken-ichi, Tanaka, c/o Hodogaya Chem. Co., Ltd. Tsukuba-shi, Ibaraki (JP)**
- **Katsuya, Yamaguchi, c/o Hodogaya Chem. Co., Ltd. Tsukuba-shi, Ibaraki (JP)**
- **Fumio, Fukuhara, c/o Hodogaya Chem. Co., Ltd. Tsukuba-shi, Ibaraki (JP)**
- **Hiroshi, Kawada, c/o Hodogaya Chem. Co., Ltd. Tsukuba-shi, Ibaraki (JP)**
- **Chieko, Inayoshi, c/o Hodogaya Chem. Co., Ltd. Tsukuba-shi, Ibaraki (JP)**
- **Kaoru, Kasahara, c/o Hodogaya Chem. Co., Ltd. Tsukuba-shi, Ibaraki (JP)**

(74) Representative:
**Hansen, Bernd, Dr. Dipl.-Chem. et al**
**Hoffmann Eitle,**
**Patent- und Rechtsanwälte,**
**Postfach 81 04 20**
**81904 München (DE)**

(56) References cited:
**EP-A- 0 328 954**
**EP-A- 0 384 211**
**EP-A- 0 509 341**
**US-A- 2 726 247**
**US-A- 4 324 899**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 566 138 B1

**Description**

FIELD OF THE INVENTION

This invention relates to thiazole derivatives which are useful as fungicides.

BACKGROUND OF THE INVENTION

The high agricultural productivity of today is ensured by the application of fertilizers, agricultural chemicals and various farm materials.

On the other hand, continuous application of agricultural chemicals causes a serious problem, namely, the appearance of chemical-resistant strains.

Therefore, it has been urgently required to develop novel compounds which are useful as a fungicide.

US-A-4 324,899 discloses 2-amino-5-cyanothiazoles and the preparation thereof. EP-A-0 384 211 describes acrylic acid ester-substituted oxazoles and thiazoles and their use as fungicides. EP-A-0 328 954 discloses 5-cyano-2,4-diaminothiazole derivatives, a process for their preparation and their use as herbicides and fungicides. US-A-2 726,247 describes 2-amino-4-trifluoromethylthiazole compounds which are intermediates for the preparation of textile dyes and pharmaceutical compounds, and a process for their preparation. EP-A-0 509 341 discloses cyanothiazole derivatives, a process for their preparation and their use as herbicides.

It is an object of the present invention to provide a novel thiazole derivative having a fungicidal action.

SUMMARY OF THE INVENTION

The present inventors have conducted extensive studies in order to develop compounds having a fungicidal action and, as a result, completed the present invention.

Accordingly, the present invention provides a thiazole derivative represented by the following formula (I):

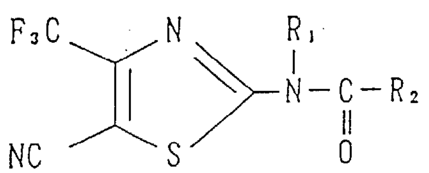

(I)

wherein $R_1$ represents a hydrogen atom or an alkyl group; and $R_2$ represents a phenyl group, a substituted phenyl group, a benzyl group, a substituted benzyl group, a phenylalkyl group, a naphthyl group, an alkyl group, a cycloalkyl group, a substituted alkyl group, an alkenyl group, an alkynyl group, a furyl group, a thienyl group, a 5-membered heterocyclic ring containing 1 to 3 nitrogen atoms in the ring and a 6-membered heterocyclic ring containing 1 to 3 nitrogen atoms in the ring; provided that when the 5-membered heterocyclic ring or the 6-membered heterocyclic ring contains one or two nitrogen atom, the remaining atoms in the ring are all carbon atoms or a combination of carbon atoms with one oxygen atom or one sulfur atom; and further provided that the furyl group, the thienyl group, the 5-membered heterocyclic ring and the 6-membered heterocyclic ring may be substituted by one to three substituents selected from an alkyl group, an alkoxy group, a halogen atom and a haloalkyl group; and a fungicide composition comprising the thiazole derivative represented by formula (I) as an active ingredient, and a carrier.

DETAILED DESCRIPTION OF THE INVENTION

The alkyl group represented by $R_1$ in formula (I) contains from 1 to 6 carbon atoms, preferably from 1 to 4 carbon atoms. Specific examples thereof include methyl, ethyl, n-propyl, i-propyl, n-butyl, i-butyl and t-butyl groups. Among them, methyl group is preferred.

The phenylalkyl group represented by $R_2$ in formula (I) contains from 7 to 15 carbon atoms, preferably from 7 to 11 carbon atoms. Specific examples thereof include 1-phenylethyl, 1-phenyl-1-methylethyl, 1-phenylpropyl, 1-phenyl-1-methylpropyl, 1-phenylbutyl, 1-phenylpentyl, 1-phenyl-1-methylbutyl and 1-phenyl-1-ethylpropyl groups. Among them, 1-phenylethyl and 1-phenyl-1-methylethyl groups are preferred.

The alkyl group represented by $R_2$ in formula (I) contains from 1 to 10 carbon atoms, preferably from 1 to 8 carbon atoms. Specific examples thereof include methyl, ethyl, n-propyl, i-propyl, n-butyl, i-butyl, t-butyl, n-pentyl, n-hexyl, n-heptyl and n-octyl groups. Among them, methyl, ethyl, i-propyl, n-butyl, i-butyl, t-butyl, n-pentyl and n-hexyl groups are

preferred.

The cycloalkyl group represented by $R_2$ in formula (I) contains from 3 to 8 carbon atoms, preferably from 3 to 6 carbon atoms. Specific examples thereof include cyclopropyl, cyclobutyl, cyclopentyl and cyclohexyl groups. Among them, cyclopropyl and cyclopentyl groups are preferred.

The alkenyl group represented by $R_2$ in formula (I) contains from 2 to 8 carbon atoms, preferably from 2 to 6 carbon atoms. Specific examples thereof include vinyl, allyl, butenyl, pentenyl and hexenyl groups. Among them, allyl group is preferred.

The alkynyl group represented by $R_2$ in formula (I) contains from 2 to 8 carbon atoms, preferably from 2 to 6 carbon atoms. Specific examples thereof include ethynyl, propynyl, butynyl, pentynyl and hexynyl groups. Among them, butynyl group is preferred.

Specific examples of the 5-membered heterocyclic ring containing 1 to 3 nitrogen atoms in the ring represented by $R_2$ in formula (I) include pyrrole, pyrroline, pyrrolidine, oxazole, isoxazole, thiazole, isothiazole, imidazole, imidazoline, imidazolidine, pyrazole, pyrazoline, pyrazolidine and triazole. Among them, pyrrole and pyrazole are preferred.

Specific examples of the 6-membered heterocyclic ring containing 1 to 3 nitrogen atoms in the ring represented by $R_2$ in formula (I) include pyridine, oxazine, pyridazine, piperazine, morpholine, pyrimidine, pyrazine, oxadiazine, thiadiazine and triazine groups. Among them, pyridine and pyrazine groups are preferred.

Examples of the substituent of the substituted phenyl, substituted benzyl and substituted alkyl groups represented by $R_2$ in formula (I) include halogen atoms (e.g., fluorine, chlorine, bromine, iodine) and hydroxyl, nitro, cyano, alkyl (e.g., methyl, ethyl, n-propyl, i-propyl, n-butyl, i-butyl, t-butyl), alkoxy (e.g., methoxy, ethoxy, n-propoxy, i-propoxy, n-butoxy, i-butoxy, t-butoxy), alkylcarbonyl (e.g., acetyl, propionyl, n-butyryl, i-butyryl), alkoxycarbonyl (e.g., methoxycarbonyl, ethoxycarbonyl, n-propoxycarbonyl, i-propoxycarbonyl), alkylcarbonyloxy (e.g., acetyloxy, propionyloxy, n-butyryloxy, i-butyryloxy), alkylamino (e.g., methylamino, ethylamino, propylamino, i-propylamino, n-butylamino, i-butylamino, t-butylamino, dimethylamino, methylethylamino, diethylamino, methylpropylamino), haloalkyl (e.g., trifluoromethyl, trichloromethyl, trifluroethyl, thrichloroethyl), haloalkoxy (e.g., trifluoromethoxy, trifluoroethoxy, trichloromethoxy, trichloroethoxy), alkylthio (e.g., methylthio, ethylthio, propylthio, i-propylthio, butylthio, i-butylthio, t-butylthio), alkylsulfonyl (e.g., methylsulfonyl, ethylsulfonyl, propylsulfonyl, i-propylsulfonyl, butylsulfonyl, i-butylsulfonyl, t-butylsulfonyl), alkoxycarbonylalkoxy (e.g., ethoxycarbonylmethoxy) and alkenyl (e.g., vinyl, allyl, butenyl), each containing 1 to 6 carbon atoms, preferably from 1 to 4 carbon atoms. Among them, methyl, ethyl, t-butyl, hydroxyl, methoxy, acetyl, methoxycarbonyl, acetyloxy, nitro, dimethylamino, cyano, trifluoromethyl, trifluoromethoxy, methylthio, methylsulfonyl, ethoxycarbonylmethoxy and vinyl groups and fluorine, chlorine and bromine atoms are preferred.

The alkyl, alkoxy and haloalkyl groups which may be substituted on the furyl or thienyl group, the 5-membered heterocyclic ring or the 6-membered heterocyclic ring represented by $R_2$ in formula (I) contain from 1 to 6 carbon atoms, preferably from 1 to 4 carbon atoms. Specific examples thereof include methyl, ethyl, n-propyl, i-propyl, n-butyl, i-butyl, t-butyl, methoxy, ethoxy, n-propoxy, i-propoxy, n-butoxy, i-butoxy, t-butoxy, trifluoromethyl, trifluoroethyl, trichloromethyl, and trichloroethyl groups. Among them, methyl, methoxy, ethoxy, and trifluoromethyl groups are preferred.

Examples of the halogen atom which may be substituted on the furyl or thienyl group, the 5-membered heterocyclic ring or the 6-membered heterocyclic ring represented by $R_2$ in formula (I) or which is contained in the haloalkyl group include fluorine, chlorine, bromine and iodine atoms.

Each of the compounds of the present invention can be prepared by the following method. Namely, a compound represented by formula (II):

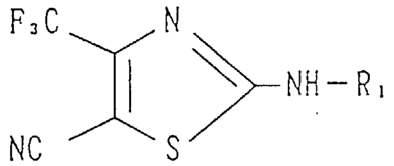

(II)

wherein $R_1$ represents the same meaning as defined above;
is reacted with a known acid halide compound represented by formula (III)

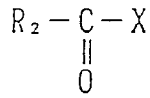

(III)

wherein $R_2$ represents the same meaning as defined above; and
X represents a halogen atom;

or a known acid anhydride compound represented by the following formula:

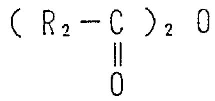

(IV)

wherein $R_2$ represents the same meaning as defined above.

In the production method as described above, the reaction is usually performed in the presence of an appropriate base. Examples of the base include organic bases such as triethylamine and pyridine and inorganic bases such as sodium methoxide, sodium hydride, potassium hydroxide, potassium carbonate and sodium hydrogencarbonate.

In the production method as described above, further, the reaction is performed either without using any solvent or in the presence of an inert solvent. Examples of usable solvents include hydrocarbons such as benzene and toluene, halogenated hydrocarbons such as chloroform and dichloroethane, ketones such as acetone and methyl ethyl ketone, ethers such as diethyl ether, tetrahydrofuran and dioxane and polar solvents such as N,N-dimethylformamide, dimethyl sulfoxide and acetonitrile.

In the production method as described above, the reaction temperature usually ranges from room temperature to the reflux temperature of the employed solvent. When it is required to shorten the reaction time, the reaction may be preferably carried out under heating.

The compound represented by formula (II), which is an intermediate in the production of the compound of formula (I) of the present invention, is also a novel compound according to the present invention and can be produced by the following method.

That is to say, 2-chloro-4-trifluoromethyl-5-thiazolecarboxylic acid (V), which can be synthesized by a known method as disclosed, for example, in J. Heterocyclic Chem., 22, 1621 (1985), is converted into an acid chloride derivative (VI) with the use of thionyl chloride and then reacted with aqueous ammonia to thereby give an amide derivative (VII). Then this compound (VII) is reacted with an appropriate dehydrating agent such as phosphorus oxychloride to thereby give 2-chloro-5-cyano-4-trifluoromethylthiazole (VIII). This compound (VIII) is then reacted with aqueous ammonia or various alkylamines in a solvent such as dioxane, tetrahydrofuran, N,N-dimethyl-formamide, dimethyl sulfoxide or acetonitrile. Thus the novel compound of formula (II) can be obtained.

The fungicide composition according to the present invention contains the thiazole derivative represented by formula (I) as an active ingredient. Since these thiazole derivatives are novel compounds, it is expected that they are effective on chemical-resistant strains. When the thiazole derivative of the present invention is to be used as an agricultural fungicide, the thiazole derivative can be mixed with various carriers and additives and formulated into various forms such as a wettable powder, an emulsifiable concentrate, a dust, granules or a flowable by techniques commonly employed in the production of agricultural chemicals. Furthermore, the thiazole derivative of the present invention is also usable as an industrial fungicide and bactericide. It exerts excellent antifungous activities on microorganisms belonging to the genus, for example, *Bacillus*, *Aspergillus*, *Penicillium* or *Saccharomyces*.

As a liquid carrier which can be used as a carrier to be employed in the agricultural fungicide composition according to the present invention, it may be selected from organic solvents commonly employed in the art, while as a solid carrier, it may be selected from clay minerals and pumice commonly employed in the art. In the formulation process, surfactants may be further added in order to impart emulsifiability, dispersibility or spreadability to the product. Furthermore, the fungicide may be blended with agricultural chemicals such as fertilizers, herbicides, insecticides or other fungicides.

In order to use as a fungicide, it is needed to apply the active ingredient in such an amount as to sufficiently achieve the desired effects. The application amount may range from 20 to 2,000 g/ha, and, in general, from 100 to 1,000 g/ha. The content of the active ingredient in the composition is adjusted to from 0.1 to 50 % by weight and the composition is formulated into a wettable powder, an emulsifiable concentrate, a dust, granules or a flowable.

An emulsifiable concentrate can be prepared by dissolving the active ingredient in an organic solvent usable for agricultural purposes and then adding an emulsifier soluble in the solvent. Examples of the solvent include xylene, o-chlorotoluene, cyclohexanone, isophorone, N,N-dimethylformamide, dimethyl sulfoxide and mixtures thereof. As particularly suitable solvents, mixtures of aromatic hydrocarbons, ketones and polar solvents may be cited. A surfactant may be used as an emulsifier in an amount of from 1 to 20 % by weight based on the emulsifiable concentrate. Either anionic, cationic or nonionic surfactants may be used therefor. The concentration of the active ingredient may range from 0.5 to 50 % by weight, preferably from 5 to 30 % by weight.

Examples of the above-mentioned surfactant include anionic surfactants such as alkyl sulfuric esters, alkyl diphenyl ether disulfonates, naphthyl methanesulfonates, ligninsulfonates, alkyl sulfosuccinates, alkyl benzenesulfonates and alkyl phosphates; cationic surfactants such as alkylamine salts and quaternary ammonium salts; and nonionic surfactants such as polyoxyethylene alkyl ethers, polyoxyethylene aryl ethers, sorbitan fatty acid esters, polyoxyethylene sorbitan fatty acid esters, polyoxyethylene sorbitol fatty acid esters, glycerol fatty acid esters and polyoxyethylene fatty acid esters.

A wettable powder may be prepared by adding the active ingredient to an inert finely pulverized solid carrier and a surfactant. In general, the content of the active ingredient ranges from 2 to 50 % by weight while the content of the surfactant ranges from 1 to 20 % by weight. As the inert finely pulverized solid carrier, native clay, silicates, silica and carbonates of alkaline earth metals may be used. Typical examples thereof include kaolin, zieclite, talc, diatomaceous earth, magnesium carbonate, calcium carbonate and dolomite. As an emulsifier, a spreader or a dispersing agent to be used therein, commonly employed anionic surfactants, nonionic surfactants and mixtures thereof may be used. As the surfactants, those employed in the emulsifiable concentrate are usable in this instance.

A dust may be prepared by blending the active ingredient with an inert carrier commonly employed in the art, for example, talc, a fine powder of clay, pyrophyllite, diatomaceous earth or magnesium carbonate. The concentration of the active ingredient may range from 0.1 to 20 % by weight, preferably from 0.5 to 5 % by weight.

Granules may be prepared by mixing the active ingredient with an inert finely pulverized carrier, for example, bentonite, diatomaceous earth, kaolin, clay or talc, kneading the obtained mixture with water and then granulating the mixture with a granulating apparatus. Alternately, it may be prepared by adhering a solution of the active ingredient and a spreading agent to a granular carrier which have been previously prepared by granulation and the granular size thereof has been adjusted to a range of from about 15-to 30-mesh or mineral granules which have been previously prepared by grinding native pumice, acid clay or zeolite and the range of the granular size thereof has been adjusted. The concentration of the active ingredient in such granules ranges from 0.2 to 20 % by weight, preferably from 1 to 10 % by weight.

A flowable may be prepared by finely pulverizing the active ingredient and mixing it with a surfactant and water. As examples of the surfactant to be used here, the anionic surfactants, the cationic ones and the nonionic ones cited above regarding the emulsifiable concentrate may be used either alone or in a combination. The surfactant is generally mixed in an amount of from 1 to 20 % by weight, while the content of the active ingredient may range from 1 to 50 % by weight, preferably from 2 to 20 % by weight.

The compound according to the present invention represented by the above-mentioned formula (I) has an excellent antifungous activity and therefore exerts effects as an agricultural fungicide on disease damage of plants caused by over a wide range of microorganisms. For example, it exerts excellent preventive effects on tomato late blight, grape downy mildew, various powdery mildew, apple black spot, wheat and barley leaf rust, rice blast, rice sheath blight, various gray molds, rice bacterial grain rot and cucumber bacterial spot. Further, the compound according to the present invention exerts not only excellent preventive effects but also excellent therapeutic effects, which enables post-infective disease control.

Typical compounds according to the present invention are listed in Table 1. Each compound will be expressed in the compound No. given in Table 1 hereinafter.

TABLE 1

| Compound No. | $-R_1$ | $-R_2$ | m.p. (°C) |
|---|---|---|---|
| 1 | −H | 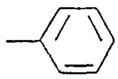 | 206~207 |
| 2 | −H | 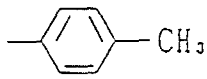 | 198~199 |
| 3 | −H | 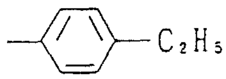 | 183~184 |
| 4 | −H | 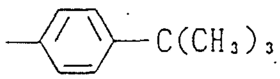 | 216~217 |
| 5 | −H | 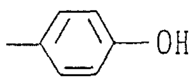 | 237~239 |
| 6 | −H | 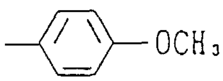 | 233~234 |
| 7 | −H | 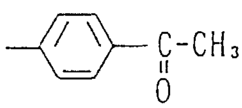 | 189~190 |
| 8 | −H | 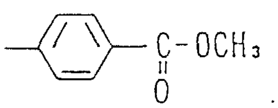 | 159~160 |
| 9 | −H | 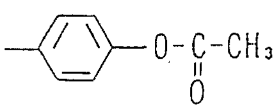 | 219~220 |
| 10 | −H | 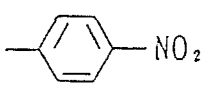 | 263°C decomposed |
| 11 | −H | 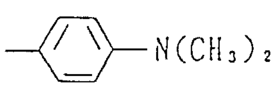 | 248~249 |

TABLE 1 (CONT'D)

| Compound No. | $-R_1$ | $-R_2$ | m.p. (℃) |
|---|---|---|---|
| 12 | −H | 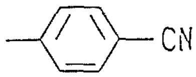 | 210∼211 |
| 13 | −H | 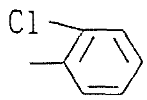 | 138∼139 |
| 14 | −H | 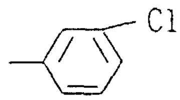 | 152∼153 |
| 15 | −H | 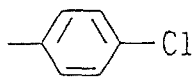 | 128∼129 |
| 16 | −H | 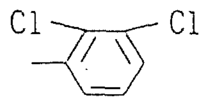 | 140∼141 |
| 17 | −H | 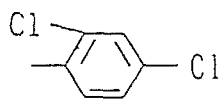 | 166∼167 |
| 18 | −H | 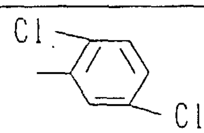 | 184∼185 |
| 19 | −H | 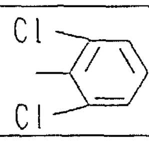 | 180∼181 |
| 20 | −H | 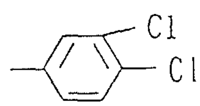 | 241∼242 |
| 21 | −H | 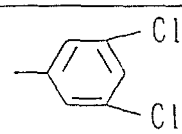 | 205∼206 |
| 22 | −H | 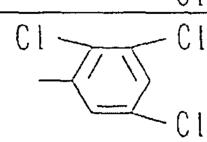 | 157∼158 |

TABLE 1 (CONT'D)

| Compound No. | $-R_1$ | $-R_2$ | m.p. (℃) |
|---|---|---|---|
| 23 | -H | 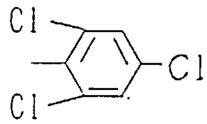 | 185~186 |
| 24 | -H | 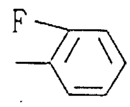 | 180~182 |
| 25 | -H | 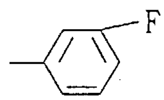 | 188~189 |
| 26 | -H | 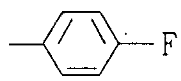 | 196~198 |
| 27 | -H | 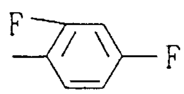 | 156~157 |
| 28 | -H | 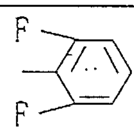 | 183~184 |
| 29 | -H | 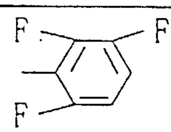 | 169~171 |
| 30 | -H | 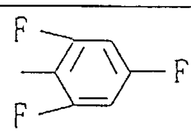 | 207~209 |
| 31 | -H | 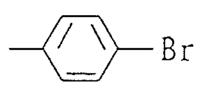 | 203~205 |
| 32 | -H | 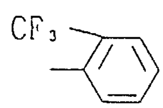 | 171~172 |
| 33 | -H | 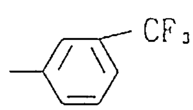 | 124~125 |

TABLE 1 (CONT'D)

| Compound No. | $-R_1$ | $-R_2$ | m.p. (℃) |
|---|---|---|---|
| 34 | $-H$ | 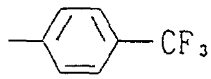 | 199~202 |
| 35 | $-H$ | 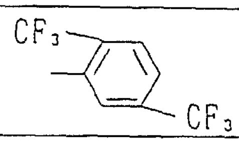 | 117~118 |
| 36 | $-H$ | 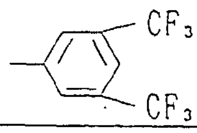 | 161~162 |
| 37 | $-H$ | 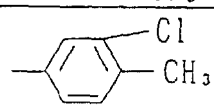 | 207~208 |
| 38 | $-H$ | 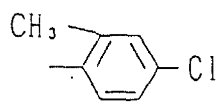 | 214~215 |
| 39 | $-H$ | 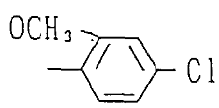 | 182~183 |
| 40 | $-H$ | 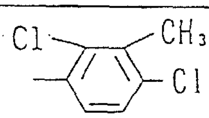 | 169~170 |
| 41 | $-H$ | 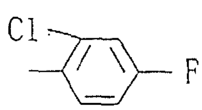 | 117~118 |
| 42 | $-H$ | 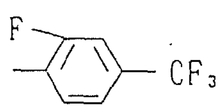 | 164~165 |
| 43 | $-H$ | 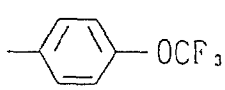 | 164~165 |
| 44 | $-H$ | 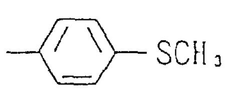 | 218~220 |

TABLE 1 (CONT'D)

| Compound No. | $-R_1$ | $-R_2$ | m.p. (℃) |
|---|---|---|---|
| 45 | −H | 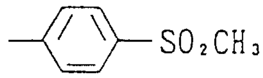 | 249~250 |
| 46 | −H | 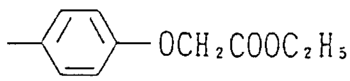 | 174~175 |
| 47 | −H | 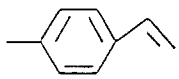 | 192~193 |
| 48 | $-CH_3$ | 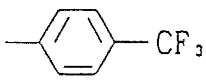 | 98~99 |
| 49 | −H | 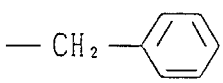 | 162~163 |
| 50 | −H | 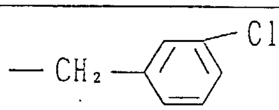 | 138~139 |
| 51 | −H | 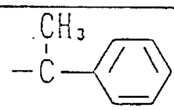 | 133~134 |
| 52 | −H | 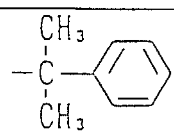 | 128~129 |
| 53 | −H | 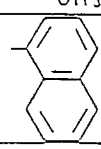 | 135~136 |
| 54 | −H | $-CH_3$ | 158~159 |
| 55 | −H | $-C_2H_5$ | 102~103 |

TABLE 1 (CONT'D)

| Compound No. | $-R_1$ | $-R_2$ | m.p. (℃) |
|---|---|---|---|
| 56 | $-H$ | $i-C_3H_7-$ | 156~157 |
| 57 | $-H$ | $n-C_4H_9-$ | 106~107 |
| 58 | $-H$ | $i-C_4H_9-$ | 124~125 |
| 59 | $-H$ | $t-C_4H_9-$ | 141~142 |
| 60 | $-H$ | $n-C_5H_{11}-$ | 90~91 |
| 61 | $-H$ | $n-C_6H_{13}-$ | 79~80 |
| 62 | $-H$ | $-CH_2CN$ | 177~178 |
| 63 | $-H$ | $-CH_2OCH_3$ | 97~98 |
| 64 | $-H$ | $-CH_2CH_2COOC_2H_5$ | 116~117 |
| 65 | $-H$ | 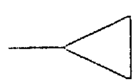 | 161~162 |
| 66 | $-H$ | 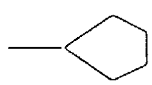 | 179~181 |

TABLE 1 (CONT'D)

| Compound No. | $-R_1$ | $-R_2$ | m.p. (℃) |
|---|---|---|---|
| 67 | -H | $-CF_3$ | 260℃ decomposed |
| 68 | -H | 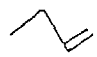 | 135~136 |
| 69 | -H | 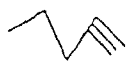 | 161~162 |
| 70 | -H | 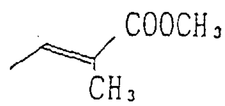 | 158~159 |
| 71 | -H | 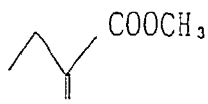 | |
| 72 | -H | 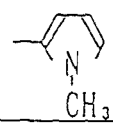 | 209~210 |
| 73 | -H | 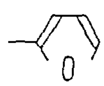 | 173~175 |
| 74 | -H | 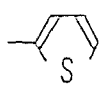 | 194~195 |
| 75 | -H | 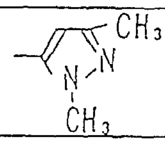 | 241~242 |
| 76 | -H | 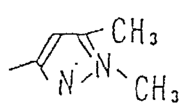 | 216~217 |
| 77 | -H | 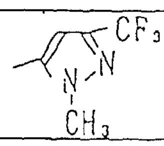 | 197~198 |

TABLE 1 (CONT'D)

| Compound No. | $-R_1$ | $-R_2$ | m.p. (°C) |
|---|---|---|---|
| 78 | $-H$ | 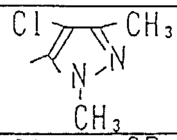 | 155~156 |
| 79 | $-H$ | 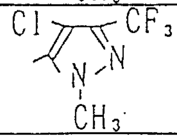 | 128~129 |
| 80 | $-H$ | 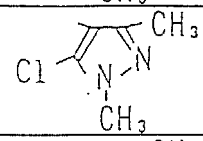 | 182~183 |
| 81 | $-H$ | 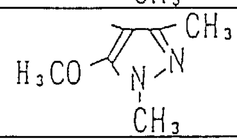 | 194~195 |
| 82 | $-H$ | 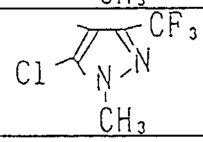 | 194~195 |
| 83 | $-H$ | 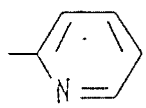 | 205~206 |
| 84 | $-H$ | 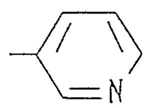 | 203~204 |
| 85 | $-H$ | 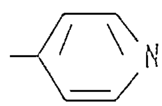 | 241~242 |
| 86 | $-H$ | 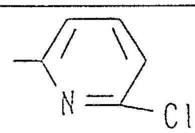 | 210~212 |
| 87 | $-H$ | 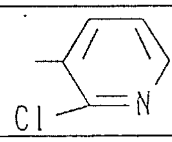 | 220~221 |
| 88 | $-H$ | 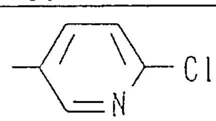 | 198~199 |

TABLE 1 (CONT'D)

| Compound No. | $-R_1$ | $-R_2$ | m.p. (°C) |
|---|---|---|---|
| 89 | -H | 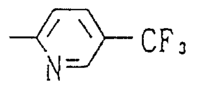 | 129~130 |
| 90 | -H | 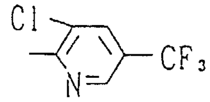 | 93~94 |
| 91 | -H | 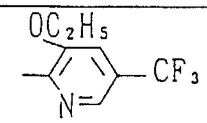 | 161~162 |
| 92 | -H | 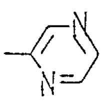 | 183~184 |

To further illustrate the present invention in greater detail, the following Examples (Synthesis Examples, Formulation Examples and Test Examples) will be given. Now typical synthesis examples of the present invention will be given.

SYNTHESIS EXAMPLE 1

Synthesis of 2-chloro-4-trifluoromethyl-5-thiazolecarbonyl chloride:

A 117.3 g portion of 2-chloro-4-trifluoromethyl-5-thiazolecarboxylic acid was dissolved in 1.1 l of dioxane. After adding 185 ml of thionyl chloride and a catalytic amount of N,N-dimethylformamide, the mixture was heated under reflux for 2.5 hours. Then the solvent was distilled off from the reaction mixture under reduced pressure. Thus 124.1 g of the target compound was obtained in the form of a colorless oily product. Yield = 98 %.

SYNTHESIS EXAMPLE 2

Synthesis of 2-chloro-4-trifluoromethyl-5-thiazolecarboxamide:

A 124.1 g portion of 2-chloro-4-trifluoromethyl-5-thiazolecarbonyl chloride was dissolved in 1.1 l of dioxane and added dropwise to 290 ml of 28 % aqueous ammonia at room temperature, followed by stirring at room temperature for 1 hour. After the completion of the reaction, the reaction mixture was neutralized with diluted hydrochloric acid, extracted with ethyl acetate, washed with water and an aqueous solution of common salt and dried over anhydrous magnesium sulfate. After distilling off the solvent under reduced pressure, the crude product thus obtained was recrystallized from a solvent mixture (toluene/hexane). Thus 98.5 g of the target compound was obtained. Yield = 86.1 %. m.p. = 89 - 90°C.

SYNTHESIS EXAMPLE 3

Synthesis of 2-chloro-5-cyano-4-trifluoromethylthiazole:

A 43.3 g portion of 2-chloro-4-trifluoromethyl-5-thiazolecarboxamide was dissolved in 160 ml of N,N-dimethylformamide and 120 ml of phosphorus oxychloride was added dropwise thereto at 40°C or below. After the completion of the addition, the resulting mixture was stirred at 70°C for 3 hours. After the completion of the reaction, the reaction mixture was poured into ice-water, neutralized with sodium carbonate and extracted with ethyl acetate. The extract was washed with water and an aqueous solution of common salt and dried over anhydrous magnesium sulfate. After

distilling off the solvent under reduced pressure, 38.2 g of the target compound was obtained in the form of a colorless oily product. Yield = 95.7 %.

SYNTHESIS EXAMPLE 4

Synthesis of 2-amino-5-cyano-4-trifluoromethylthiazole:

A 1.0 g portion of 2-chloro-5-cyano-4-trifluoromethylthiazole was dissolved in 40 ml of tetrahydrofuran and 9 ml of 28 % aqueous ammonia was added dropwise thereto. After the completion of the addition, the resulting mixture was stirred at room temperature for 8 hours. After the completion of the reaction, the reaction mixture was poured into water, neutralized with diluted hydrochloric acid and extracted with ethyl acetate. The extract was washed with water and an aqueous solution of common salt and dried over anhydrous magnesium sulfate. After distilling off the solvent under reduced pressure, the crude product thus obtained was recrystallized from a solvent mixture (toluene/hexane) to thereby give 0.82 g of the target product. Yield = 90.3 %. m.p. = 166 - 167°C.

SYNTHESIS EXAMPLE 5

Synthesis of 5-cyano-2-methylamino-4-trifluoromethylthiazole:

A 1.2 g portion of 2-chloro-5-cyano-4-trifluoromethylthiazole was dissolved in 40 ml of tetrahydrofuran and 1.32 g of a 40 % aqueous solution of methylamine was added dropwise thereto. After the completion of the addition, the resulting mixture was stirred at room temperature for 2 hours. After the completion of the reaction, the reaction mixture was poured into water, neutralized with diluted hydrochloric acid and extracted with ethyl acetate. The extract was washed with water and an aqueous solution of common salt and dried over anhydrous magnesium sulfate. After distilling off the solvent under reduced pressure, the crude product thus obtained was recrystallized from a solvent mixture (toluene/hexane) to thereby give 1.11 g of the target compound. Yield = 90.3 %. m.p. = 125 - 126°C.

SYNTHESIS EXAMPLE 6

Synthesis of N-(5-cyano-4-trifluoromethylthiazol-2-yl)-4-chlorobenzamide (Compound No. 15):

A 0.2 g portion of 2-amino-5-cyano-4-trifluoromethylthiazole was dissolved in 30 ml of acetone and 0.2 g of 4-chlorobenzoyl chloride and 0.3 g of potassium carbonate were added thereto, followed by stirring at room temperature for 3 hours. After the completion of the reaction, the reaction mixture was poured into ice-water, extracted with ethyl acetate, washed with an aqueous solution of sodium carbonate, diluted hydrochloric acid, water and an aqueous solution of common salt and then dried over anhydrous magnesium sulfate. After distilling off the solvent under reduced pressure, the crude product thus obtained was purified by silica gel column chromatography. Thus 0.21 g of the target compound was obtained. Yield = 63.5 %. m.p. = 128 - 129°C.

SYNTHESIS EXAMPLE 7

Synthesis of N-(5-cyano-4-trifluoromethylthiazol-2-yl)-propionamide: (Compound No. 55):

A 0.8 g portion of 2-amino-5-cyano-4-trifluoromethylthiazole was dissolved in 50 ml of dioxane and 0.8 ml of propionyl chloride and 1.2 ml of triethylamine were added thereto, followed by stirring at 60°C for 6 hours. After the completion of the reaction, the reaction mixture was poured into ice-water, extracted with ethyl acetate, washed with an aqueous solution of sodium carbonate, diluted hydrochloric acid, water and an aqueous solution of common salt and dried over anhydrous magnesium sulfate. After distilling off the solvent under reduced pressure, the crude product thus obtained was purified by silica gel column chromatography. Thus 0.68 g of the target compound was obtained. Yield = 74.6 %. m.p. = 102 - 103°C.

SYNTHESIS EXAMPLE 8

Synthesis of N-(5-cyano-4-trifluoromethylthiazol-2-yl)-trifluoroacetamide (compound No. 67):

A 0.8 g portion of 2-amino-5-cyano-4-trifluoromethylthiazole was dissolved in 50 ml of dioxane and 0.7 ml of trifluoroacetic anhydride and 0.7 ml of triethylamine were added thereto, followed by stirring at room temperature for 2 hours. After the completion of the reaction, the reaction mixture was poured into ice-water, extracted with ethyl acetate,

washed with an aqueous solution of sodium carbonate, diluted hydrochloric acid, water and an aqueous solution of common salt and dried over anhydrous magnesium sulfate. After distilling off the solvent under reduced pressure, the crude product thus obtained was recrystallized from a solvent mixture (ethyl acetate/hexane) to thereby give 1.03 g of the target compound. Yield = 86.0 %. Decomposed at 260°C.

SYNTHESIS EXAMPLE 9

Synthesis of N-(5-cyano-4-trifluoromethylthiazol-2-yl)-N-methyl-4-trifluoromethylbenzamide (Compound No. 48):

A 1.1 g portion of 5-cyano-2-methylamino-4-trifluoromethylthiazole was dissolved in 50 ml of dioxane and 1.6 ml of 4-trifluoromethylbenzoyl chloride and 1.5 ml of triethylamine were added thereto, followed by stirring at 60°C for 2 hours. After the completion of the reaction, the reaction mixture was poured into ice-water, extracted with ethyl acetate, washed with an aqueous solution of sodium carbonate, diluted hydrochloric acid, water and an aqueous solution of common salt and dried over anhydrous magnesium sulfate. After distilling off the solvent under reduced pressure, the crude product thus obtained was recrystallized from a solvent mixture (toluene/hexane) to thereby give 1.10 g of the target compound. Yield = 54.1 %. m.p. = 98 - 99°C.

SYNTHESIS EXAMPLE 10

Synthesis of N-(5-cyano-4-trifluoromethylthiazol-2-yl)-cyclopentanecarboxamide (Compound No. 66):

A 0.8 g portion of 2-amino-5-cyano-4-trifluoromethylthiazole was dissolved in 50 ml of dioxane and 1.2 g of cyclopentanecarbonyl chloride and 1.5 ml of triethylamine were added thereto, followed by stirring at 60°C for 6 hours. After the completion of the reaction, the reaction mixture was poured into ice-water, extracted with ethyl acetate, washed with an aqueous solution of sodium carbonate, diluted hydrochloric acid, water and an aqueous solution of common salt and dried over anhydrous magnesium sulfate. After distilling off the solvent under reduced pressure, the crude product thus obtained was purified by silica gel column chromatography to thereby give 0.78 g of the target compound. Yield = 65.1 %. m.p. = 179 - 181°C.

SYNTHESIS EXAMPLE 11

Synthesis of N-(5-cyano-4-trifluoromethylthiazol-2-yl)-1-naphtoamide (Compound No. 53):

A 0.7 g portion of 5-cyano-2-methylamino-4-trifluoromethylthiazole was dissolved in 40 ml of dioxane and 1.4 g of 1-naphthoyl chloride and 1.3 ml of triethylamine were added thereto, followed by stirring at 60°C for 4 hours. After the completion of the reaction, the reaction mixture was poured into ice-water, extracted with ethyl acetate, washed with an aqueous solution of sodium carbonate, diluted hydrochloric acid, water and an aqueous solution of common salt, dried over anhydrous magnesium sulfate, and distilled under reduced pressure. The crude product thus obtained was recrystallized from a mixed solvent (toluene/hexane) to thereby give 0.78 g of the target compound. Yield = 62.0 %. m.p. = 135 - 136°C.

SYNTHESIS EXAMPLE 12

Synthesis of N-(5-cyano-4-trifluoromethylthiazol-2-yl)-2-thiophencarboxamide (Compound No. 74):

A 0.7 g portion of 2-amino-5-cyano-4-trifluoromethylthiazole was dissolved in 40 ml of dioxane and 1.1 g of 2-thiphencarbonyl chloride and 1.3 ml of triethylamine were added thereto, followed by stirring at 60°C for 4 hours. After the completion of the reaction, the reaction mixture was poured into ice-water, extracted with ethyl acetate, washed with an aqueous solution of sodium carbonate, diluted hydrochloric acid, water and an aqueous solution of common salt, dried over anhydrous magnesium sulfate, and distilled under reduced pressure. The crude product thus obtained was purified by silica gel column chromatography to thereby give 1.13 g of the target compound. Yield = 81.3 %. m.p. = 194 - 195°C.

SYNTHESIS EXAMPLE 13

Synthesis of N-(5-cyano-4-trifluoromethylthiazol-2-yl)-4-chloro-1-methyl-3-trifluoromethyl-5-pyrazolecarboxamide (Compound No. 79):

A 1.5 g portion of 2-amino-5-cyano-4-trifluoromethylthiazole was dissolved in 40 ml of pyridine and 3.8 g of 4-chloro-1-methyl-3-trifluoromethyl-5-pyrazolecarbonyl chloride was added thereto, followed by stirring at room temperature for 2 hours. After the completion of the reaction, the reaction mixture was poured into ice-water, extracted with ethyl acetate, washed with an aqueous solution of sodium carbonate, diluted hydrochloric acid, water and an aqueous solution of common salt, dried over anhydrous magnesium sulfate, and distilled under reduced pressure. The crude product thus obtained was purified by silica gel column chromatography to thereby give 1.86 g of the target compound. Yield = 70.5 %. m.p. = 128 - 129°C.

SYNTHESIS EXAMPLE 14

Synthesis of N-(5-cyano-4-trifluoromethylthiazol-2-yl)-2-pyridinecarboxamide (Compound No. 83):

A 1.6 g portion of 2-amino-5-cyano-4-trifluoromethylthiazole was dissolved in 30 ml of pyridine and 3.5 g of 2-pyridinecarybonyl chloride was added thereto, followed by stirring at room temperature for 2 hours. After the completion of the reaction, the reaction mixture was poured into ice-water, extracted with ethyl acetate, washed with diluted hydrochloric acid, water and an aqueous solution of common salt, dried over anhydrous magnesium sulfate, and distilled under reduced pressure. The crude product thus obtained was purified by silica gel column chromatography to thereby give 1.42 g of the target compound. Yield = 57.5 %. m.p. = 205 - 206°C.

SYNTHESIS EXAMPLE 15

Synthesis of N-(5-cyano-4-trifluoromethylthiazol-2-yl)-3-chloro-5-trifluoromethyl-2-pyridinecarboxamide (Compound No. 90):

A 0.8 g portion of 2-amino-5-cyano-4-trifluoromethylthiazole was dissolved in 30 ml of dioxane and 1.0 g of 3-chloro-5-trifluoromethyl-2-pyridinecarbonyl chloride and 0.6 ml of triethylamine were added thereto, followed by stirring at 60°C for 3 hours. After the completion of the reaction, the reaction mixture was poured into ice-water, extracted with ethyl acetate, washed with diluted hydrochloric acid, water and an aqueous solution of common salt, dried over anhydrous magnesium sulfate, and distilled under reduced pressure. The crude product thus obtained was purified by silica gel column chromatography to thereby give 0.98 g of the target compound. Yield = 59.0 %. m.p. = 93 - 94°C.

SYNTHESIS EXAMPLE 16

Synthesis of N-(5-cyano-4-trifluoromethylthiazol-2-yl)-2-pyrazinecarboxamide (Compound No. 92):

A 1.2 g portion of 2-amino-5-cyano-4-trifluoromethylthiazole was dissolved in 30 ml of pyridine and 1.8 g of 2-pyrazinecarbonyl chloride was added thereto, followed by stirring at room temperature for 4 hours. After the completion of the reaction, the reaction mixture was poured into ice-water, extracted with ethyl acetate, washed with diluted hydrochloric acid, water and an aqueous solution of common salt, dried over anhydrous magnesium sulfate, and distilled under reduced pressure. The crude product thus obtained was purified by silica gel column chromatography to thereby give 1.56 g of the target compound. Yield = 83.9%. m.p. = 183 - 184°C.

Next, the compositions of the present invention will be described in greater detail by citing a formulation example for each type.

FORMULATION EXAMPLE 1 (Emulsifiable Concentrate)

| Component | Amount (parts by weight) |
|---|---|
| Compound No. 12 | 20 |
| o-Chlorotoluene | 40 |
| Cyclohexanone | 35 |
| Sorpol 900 B | 5 |

(continued)

| FORMULATION EXAMPLE 1 (Emulsifiable Concentrate) | |
|---|---|
| Component | Amount (parts by weight) |
| (Sorpol 900 B: a trademark of a product comprising a polyoxyethylenealkylphenol polymer, a polyoxyethylene alkylaryl ether, a polyoxyethylene sorbitan fatty acid ester and an anionic surfactant; manufactured by Toho Chemical Industry Co., Ltd.) | |

These components were uniformly mixed and dissolved to thereby give an emulsifiable concentrate according to the present invention.

| FORMULATION EXAMPLE 2 (Wettable Powder) | |
|---|---|
| Component | Amount (parts by weight) |
| Compound No. 4 | 25 |
| Kaolin clay | 57 |
| Sorpol 5039 | 9 |
| Sorpol 5060 | 9 |
| (Sorpol 5039: a trademark of a product comprising an ammonium polyoxyethylene alkylaryl ether sulfate; Sorpol 5060: a trademark of a product comprising a sodium alkylallylsulfonate and phosphoric acid; each manufactured by Toho Chemical Industry Co., Ltd.) | |

These components were mixed and ground to thereby give a wettable powder according to the present invention.

| FORMULATION EXAMPLE 3 (Dust) | |
|---|---|
| Component | Amount (parts by weight) |
| Compound No. 19 | 5 |
| Kaolin clay | 95 |

These components were mixed and ground to thereby give a dust according to the present invention.

| FORMULATION EXAMPLE 4 (Granule) | |
|---|---|
| Component | Amount (parts by weight) |
| Compound No. 46 | 6 |
| Bentonite | 44 |
| Talc | 44 |
| Sodium ligninsulfonate | 6 |

These components were uniformly mixed and ground. After adding water, the mixture was kneaded, granulated and dried to thereby give granules according to the present invention.

| FORMULATION EXAMPLE 5 (Flowable) | |
|---|---|
| Component | Amount (parts by weight) |
| Compound No. 24 | 10 |
| Ethylene glycol | 6 |
| Sorpol 3078 | 6 |
| Sorpol 7512 | 1 |
| Water | 77 |
| (Sorpol 3078: a trademark of a product comprising a polyoxyethylene alkylphenyl ether sulfate; Sorpol 7512: a trademark of a product; each manufactured by Toho Chemical Industry Co., Ltd.) | |

These components were uniformly mixed and ground to thereby give a flowable according to the present invention.

| FORMULATION EXAMPLE 6 (Emulsifiable Concentrate) | |
|---|---|
| Component | Amount (parts by weight) |
| Compound No. 38 | 10 |
| Isophorone | 22 |
| o-Chlorotoluene | 28 |
| Xylene | 22 |
| Sorpol 900A | 9 |
| Sorpol 900B | 9 |
| (Sorpol 900 A: a trademark of a product comprising a polyoxyethylenealkylphenol polymer, a polyoxyethylene alkylaryl ether, a polyoxyethylene sorbitan fatty acid ester and an anionic surfactant; manufactured by Toho Chemical Industry Co., Ltd.) | |

These components were uniformly mixed and dissolved to thereby give an emulsifiable concentrate according to the present invention.

To further illustrate the preventive effects of the compounds of the present invention on fungi, the following Test Examples will be given.

TEST EXAMPLE 1

Test of preventive effect on rice blast:

Rice plants (variety: *koshihikari*) were cultivated in plastic pots (diameter: 7 cm). At the difoliate stage, 10 ml portions of test compound solutions comprising 100 ppm of the test compositions prepared in accordance with the above Formulation Example 1 were sprayed with the use of a spray gun. Regarding each test compound, four lots each having 10 plants were employed, and two of them were treated with the test compound (test lot), and the other two of them were not treated with the test compound (control lot). One day after the treatment with the composition, each rice plant was inoculated with a conidium suspension of *Pyricularia oryzae* which causes rice blast. After the completion of the inoculation, the rice plants were cultivated under moisture-saturation at 25°C in the dark for 24 hours followed by at 25°C for 6 days with 12-hour photoperiod. On the 7th day after the inoculation, the lesions of the first and second leaves were examined and the preventive value was calculated in accordance with the following Equation (1). Table 2 summarizes the results.

Equation (1):

$$\text{Prevention value} = \frac{\text{(Lesions of control lot) - (Lesions of test lot)}}{\text{(Lesions of control lot)}} \times 100$$

TABLE 2

| (Preventive effect on rice blast) | |
|---|---|
| Compound No. | Preventive value |
| 2 | 100 |
| 6 | 100 |
| 9 | 100 |
| 12 | 100 |
| 19 | 100 |
| 24 | 100 |
| 31 | 100 |
| 36 | 100 |
| 41 | 100 |
| 43 | 100 |
| 47 | 100 |

TABLE 2 (continued)

| (Preventive effect on rice blast) | |
|---|---|
| Compound No. | Preventive value |
| 49 | 100 |
| 54 | 100 |
| 56 | 100 |
| 57 | 100 |
| 59 | 100 |
| 63 | 100 |
| 66 | 100 |
| 68 | 100 |
| 84 | 100 |
| 90 | 100 |

TEST EXAMPLE 2

Test of preventive effect on wheat powdery mildew:

Wheat plants (variety: *norin No. 61*) were cultivated in plastic pots (diameter: 7 cm). At the difoliate stage, 10 ml portions of test compound solutions comprising 100 ppm of the test compositions prepared in accordance with the above Formulation Example 2 were sprayed with the use of a spray gun. Regarding each test compound, four lots each having 13 plants were employed, and two of them were treated with the test compound (test lot), whereas the other two of them were not treated with the test compound (control lot). One day after the treatment with the composition, each wheat plant was inoculated with a conidium suspension of *Erysiphe graminis* which causes wheat powdery mildew. After the completion of the inoculation, the wheat plants were cultivated at 20°C in the dark for 12 hours followed by at 20°C for 6 days with 12-hour photoperiod. On the 7th day after the inoculation, the rate of the lesions of the first and second leaves were examined, and the preventive value was calculated in accordance with Equation (1). Table 3 summarizes the results.

TABLE 3

| (Preventive effect on wheat powdery mildew) | |
|---|---|
| Compound No. | Preventive value |
| 1 | 100 |
| 4 | 100 |
| 13 | 100 |
| 17 | 100 |
| 32 | 100 |
| 55 | 100 |
| 58 | 100 |
| 60 | 100 |
| 65 | 100 |
| 67 | 100 |

TEST EXAMPLE 3

Test of preventive effect on wheat leaf rust:

Wheat plants (variety: *norin No. 61*) were cultivated in plastic pots (diameter: 7 cm). At the difoliate stage, 10 ml portions of test compound solutions comprising 100 ppm of the test compositions prepared in accordance with the above Formulation Example 6 were sprayed with the use of a spray gun. Regarding each test compound, four lots each having 13 plants were employed, and two of them were treated with the test compound (test lot), whereas the other two of them were not treated with the test compound (control lot). One day after the treatment with the composition, each wheat plant was inoculated with a conidium suspension of *Puccinia recondita* which causes wheat leaf rust. After

the completion of the inoculation, the wheat plants were cultivated under moisture-saturation at 20°C in the dark for 24 hours followed by at 20°C for 6 days with 12-hour photoperiod. On the 7th day after the inoculation, the rate of the lesions of the first and second leaves were examined, and the preventive value was calculated in accordance with Equation (1). Table 4 summarizes the results.

TABLE 4

| (Preventive effect on wheat leaf rust) | |
|---|---|
| Compound No. | Preventive value |
| 23 | 100 |
| 27 | 100 |
| 28 | 100 |
| 30 | 100 |
| 80 | 100 |

TEST EXAMPLE 4

Test of preventive effect on wheat glume blotch:

Wheat plants (variety: *norin No. 61*) were cultivated in plastic pots (diameter: 7 cm). At the difoliate stage, 10 ml portions of test compound solutions comprising 200 ppm of the test compositions prepared in accordance with the above Formulation Example 5 were sprayed with the use of a spray gun. Regarding each test compound, four lots each having 13 plants were employed, and two of them were treated with the test compound (test lot), whereas the other two of them were not treated with the test compound (control lot). One day after the treatment with the composition, each wheat plant was inoculated with a conidium suspension of *Leptosphaeria nodorum* which causes wheat glume blotch. After the completion of the inoculation, the wheat plants were cultivated under moisture-saturation at 20°C in the dark for 48 hours followed by at 20°C for 6 days with 12-hour photoperiod. On the 7th day after the inoculation, the rate of the lesions of the first and second leaves were examined, and the preventive value was calculated in accordance with Equation (1). Table 5 summarizes the results.

TABLE 5

| (Preventive effect on wheat glume blotch) | |
|---|---|
| Compound No. | Preventive value |
| 48 | 100 |
| 53 | 100 |
| 70 | 100 |

The compounds of the present invention are highly usable as an agricultural fungicide and an industrial fungicide.

**Claims**

**1.** A thiazole derivative represented by the following formula (I):

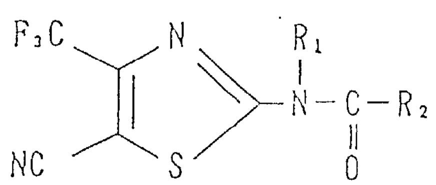

(I)

wherein $R_1$ represents a hydrogen atom or an alkyl group; and $R_2$ represents a phenyl group, a benzyl group or an alkyl group, each optionally substituted with one or more selected from halogen, hydroxyl, nitro, cyano, $C_{1-6}$-alkyl, $C_{1-6}$-alkoxy, $C_{1-6}$-alkylcarbonyl, $C_{1-6}$-alkoxycarbonyl, $C_{1-6}$-alkylcarbonyloxy, $C_{1-6}$-alkylamino, $C_{1-6}$-haloalkyl,

$C_{1-6}$-haloalkoxy, $C_{1-6}$-alkylthio, $C_{1-6}$-alkylsulfonyl, $C_{1-6}$-alkoxycarbonylalkoxy and $C_{1-6}$-alkenyl, a phenylalkyl group, a naphthyl group, a cycloalkyl group, an alkenyl group, an alkynyl group, a furyl group, a thienyl group, a 5-membered heterocyclic ring containing 1 to 3 nitrogen atoms in the ring and a 6-membered heterocyclic ring containing 1 to 3 nitrogen atoms in the ring; provided that when the 5-membered heterocyclic ring or the 6-membered heterocyclic ring contains one or two nitrogen atom, the remaining atoms in the ring are all carbon atoms or a combination of carbon atoms with one oxygen atom or one sulfur atom; and further provided that the furyl group, the thienyl group, the 5-membered heterocyclic ring and the 6-membered heterocyclic ring may be substituted by one to three substituents selected from an alkyl group, an alkoxy group, a halogen atom and a haloalkyl group.

**2.** The thiazole derivative of claim 1, which is selected from N-(5-cyano-4-trifluoromethylthiazol-2-yl)-4-chlorobenzamide, N-(5-cyano-4-trifluoromethylthiazol-2-yl)propionamide, N-(5-cyano-4-trifluoromethylthiazol-2-yl)trifluoroacetamide, N-(5-cyano-4-trifluoromethylthiazol-2-yl)-N-methyl-4-trifluoromethylbenzamide, N-(5-cyano-4-trifluoromethylthiazol-2-yl)-cyclopentanecarboxamide, N-(5-cyano-4-trifluoromethylthiazol-2-yl)-1-naphtoamide, N-(5-cyano-4-trifluoromethylthiazol-2-yl)-2-thiophencarboxamide, N-(5-cyano-4-trifluoromethylthiazol-2-yl)-4-chloro-1-methyl-3-trifluoromethyl-5-pyrazolecarboxamide, N-(5-cyano-4-trifluoromethylthiazol-2-yl)-2-pyridinecarboxamide, N-(5-cyano-4-trifluoromethylthiazol-2-yl)-3-chloro-5-trifluoromethyl-2-pyridinecarboxamide, N-(5-cyano-4-trifluoromethylthiazol-2-yl)-2-pyrazinecarboxamide.

**3.** A fungicide composition comprising a thiazole derivative according to claim 1; and a carrier.

**4.** A process for the preparation of a thiazole derivative according to claim 1 comprising the reaction of a compound of the formula (II):

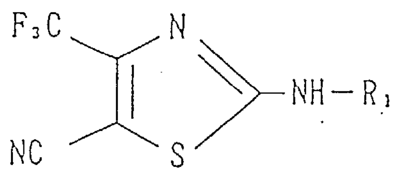

(II)

wherein $R_1$ represents the same meaning as defined in claim 1;
with an acid halide compound represented by the formula (III):

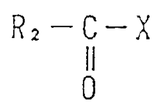

(III)

wherein $R_2$ represents the same meaning as defined in claim 1; and X represents a halogen atom
or with an acid anhydride compound represented by the following formula (IV):

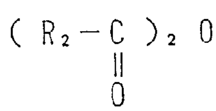

(IV)

wherein $R_2$ represents the same meaning as defined in claim 1.

**Patentansprüche**

**1.** Thiazolderivat gemäß der folgenden Formel (I):

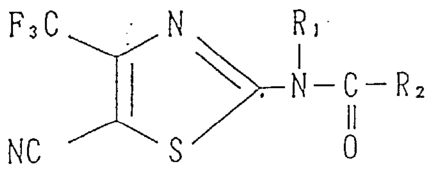

(I)

worin $R_1$ ein Wasserstoffatom oder eine Alkylgruppe darstellt; und $R_2$ eine Phenylgruppe, eine Benzylgruppe oder eine Alkylgruppe darstellt, die jeweils gegebenenfalls mit einer oder mehreren Gruppen, ausgewählt aus Halogen-, Hydroxyl-, Nitro-, Cyano-, $C_{1-6}$-Alkyl-, $C_{1-6}$-Alkoxy-, $C_{1-6}$-Alkylcarbonyl-, $C_{1-6}$-Alkoxycarbonyl-, $C_{1-6}$-Alkylcarbonyloxy-, $C_{1-6}$-Alkylamino-, $C_{1-6}$-Halogenalkyl-, $C_{1-6}$-Halogenalkoxy-, $C_{1-6}$-Alkylthio-, $C_{1-6}$-Alkylsulfonyl-, $C_{1-6}$-Alkoxycarbonylalkoxy- und $C_{1-6}$-Alkenyl-, einer Phenylalkylgruppe, einer Naphthylgruppe, einer Cycloalkylgruppe, einer Alkenylgruppe, einer Alkinylgruppe, einer Furylgruppe, einer Thienylgruppe, einem 5-gliedrigen heterocyclischen Ring, der 1 bis 3 Ringstickstoffatome enthält, und einem 6-gliedrigen heterocyclischen Ring, der 1 bis 3 Ringstickstoffatome enthält, substituiert sind; unter der Voraussetzung, daß wenn der 5-gliedrige heterocyclische Ring oder der 6-gliedrige heterocyclische Ring ein oder zwei Stickstoffatome enthält, die verbleibenden Ringatome alle Kohlenstoffatome oder eine Kombination von Kohlenstoffatomen mit einem Sauerstoffatom oder einem Schwefelatom sind; und unter der weiteren Voraussetzung, daß die Furylgruppe, die Thienylgruppe, der 5-gliedrige heterocyclische Ring und der 6-gliedrige heterocyclische Ring mit einem bis drei Substituenten, ausgewählt aus einer Alkylgruppe, einer Alkoxygruppe, einem Halogenatom und einer Halogenalkylgruppe, substituiert sein können.

**2.** Thiazolderivat gemäß Anspruch 1, ausgewählt aus N-(5-Cyano-4-trifluormethylthiazol-2-yl)-4-chlorbenzamid, N-(5-Cyano-4-trifluormethylthiazol-2-yl)-propionamid, N-(5-Cyano-4-trifluormethylthiazol-2-yl)-trifluoracetamid, N-(5-Cyano-4-trifluormethylthiazol-2-yl)-N-methyl-4-trifluormethylbenzamid, N-(5-Cyano-4-trifluormethylthiazol-2-yl)-cyclopentancarboxamid, N-(5-Cyano-4-trifluormethylthiazol-2-yl)-1-naphthoamid, N-(5-Cyano-4-trifluormethylthiazol-2-yl)-2-thiophencarboxamid, N-(5-Cyano-4-trifluormethylthiazol-2-yl)-4-chlor-1-methyl-3-trifluormethyl-5-pyrazolcarboxamid, N-(5-Cyano-4-trifluormethylthiazol-2-yl)-2-pyridincarboxamid, N-(5-Cyano-4-trifluormethylthiazol-2-yl)-3-chlor-5-trifluormethyl-2-pyridincarboxamid, N-(5-Cyano-4-trifluormethylthiazol-2-yl)-2-pyrazincarboxamid.

**3.** Fungizide Zusammensetzung, die ein Thiazolderivat gemäß Anspruch 1 und einen Träger umfaßt.

**4.** Verfahren zur Herstellung eines Thiazolderivates gemäß Anspruch 1, das die Reaktion einer Verbindung der Formel (II):

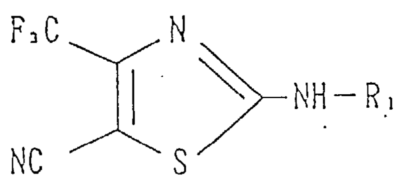

(II)

worin $R_1$ dieselbe Bedeutung wie in Anspruch 1 definiert aufweist; mit einer Säurehalogenidverbindung gemäß Formel (III):

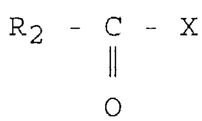

(III)

worin $R_2$ die gleiche Bedeutung wie in Anspruch 1 definiert, aufweist; und X ein Halogenatom darstellt oder mit einer Säureanhydridverbindung gemäß der folgenden Formel (IV) :

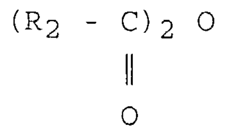

(IV)

worin $R_2$ die gleiche Bedeutung, wie in Anspruch 1 definiert, aufweist, umfaßt.

## Revendications

1. Un dérivé de thiazole représenté par la formule suivante (I):

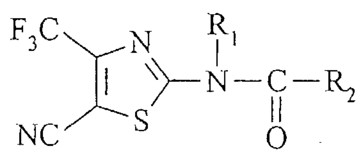

(I)

dans laquelle $R_1$ représente un atome d'hydrogène ou un groupe alkyle ; et $R_2$ représente un groupe phényle, un groupe benzyle ou un groupe alkyle; chacun étant facultativement substitué par un ou plusieurs substituants choisis parmi les atomes d'halogène, les groupes hydroxy, nitro, cyano, alkyle en $C_{1-6}$, alcoxy en $C_{1-6}$, (alkyl en $C_{1-6}$) carbonyle, (alcoxy en $C_{1-6}$)carbonyle, (alkyl en $C_{1-6}$)carbonyloxy, (alkyl en $C_{1-6}$)amino, halogénoalkyle en $C_{1-6}$, halogénoalcoxy en $C_{1-6}$, (alkyl en $C_{1-6}$)thio, (alkyl en $C_{1-6}$) sulfonyle, alcoxycarbonylalcoxy en $C_{1-6}$ et alcényle en $C_{1-6}$, un groupe phénylalkyle, un groupe naphtyle, un groupe cycloalkyle, un groupe alcényle, un groupe alcynyle, un groupe furyle, un groupe thiényle, un groupe hétérocyclique à 5 chaînons contenant 1 à 3 atomes d'azote dans le cycle et un groupe hétérocyclique à 6 chaînons contenant 1 à 3 atomes d'azote dans le cycle ; à condition que lorsque le groupe hétérocyclique à 5 chaînons ou le groupe hétérocyclique à 6 chaînons contient un ou deux atomes d'azote, les autres atomes dans le cycle sont tous des atomes de carbone ou une combinaison d'atomes de carbone avec un atome d'oxygène ou un atome de soufre ; et de plus à condition que le groupe furyle, le groupe thiényle, le groupe hétérocyclique à 5 chaînons et le groupe hétérocyclique à 6 chaînons puissent être substitués par un à trois substituants, choisis parmi un groupe alkyle, un groupe alcoxy, un atome d'halogène et un groupe halogénoalkyle.

2. Dérivé de thiazole selon la revendication 1, qui est choisi parmi le N-(5-cyano-4-trifluorométhylthiazol-2-yl)-4-chlorobenzamide, le N-(5-cyano-4-trifluorométhylthiazol-2-yl)propionamide, le N-(5 -cyano-4-trifluorométhylthiazol-2-yl)trifluoroacétamide, le N-(5-cyano-4-trifluorométhylthiazol-2-yl)-N-méthyl-4-trifluorométhylbenzamide, le N-(5-cyano-4-trifluorométhylthiazol-2-yl)cyclopentanecarboxamide, le N-(5-cyano-4-trifluorométhylthiazol-2-yl)-1-naphtylamide, le N-(5-cyano-4-trifluorométhylthiazol-2-yl)-2-thiophénecarboxamide, le N-(5-cyano-4-trifluorométhylthiazol-2-yl)-4-chloro-1-méthyl-3 -trifluorométhyl-5 -pyrazolecarboxamide, le N-(5-cyano-4-trifluorométhylthiazol-2-yl)-2-pyridinecarboxamide, le N-(5 -cyano-4-trifluorométhylthiazol-2-yl)-3 -chlore-5-trifluorométhyl-2-pyridinecarboxamide, le N-(5-cyano-4-trifluorométhylthiazol-2-yl)-2-pyrazinecarboxamide.

3. Composition fongicide comprenant un dérivé de thiazole selon la revendication 1 et un excipient.

4. Procédé pour la préparation d'un dérivé de thiazole selon la revendication 1, comprenant la réaction d'un composé de formule (II):

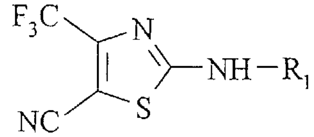

(II)

dans laquelle $R_1$ possède la même signification que celle définie dans la revendication 1 ; avec un composé halogénure d'acide représenté par la formule (III):

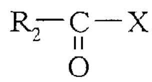 (III)

dans laquelle $R_2$ possède la même signification que celle définie dans la revendication 1 ; et X représente un atome d'halogène, ou avec un composé anhydride d'acide représenté par la formule suivante (IV) :

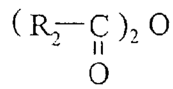 (IV)

dans laquelle $R_2$ possède la même signification que celle définie dans la revendication 1.